# EUROPEAN PATENT APPLICATION

(11) **EP 1 655 046 A1**
(43) Date of publication of application: **10.05.2006**
(21) Application number: 04026304.8
(22) Date of filing: 05.11.2004
(51) Int. Cl.: A61M 5/32

(54) **Safe syringe**

(71) Applicant: Wu, Wei-Shui, Taichung Hsien (TW)
(72) Inventor: Wu, Wei-Shui, Taichung Hsien (TW)
(74) Representative: Viering, Jentschura & Partner

(57) **Abstract**

A safe syringe mainly includes a tube (1), a push rod (2), a needle seat (3), and a needle lid (4). The needle seat passes through a round tube (11) of the syringe tube via a base (31) and pushing against a bottom of an annular protruding portion (111) along two sides of a major axis (312) of a plane (314) on the base to form a resistance in a container portion (12). After injection of a medicament, a protruding pillar (25) on the rod enters the base of the seat, thereby the base gradually forming a cone and the cone of pillar and a connection portion of the base is made to combine by a tension and a friction. After combination, an external diameter of the base is still slightly smaller than an internal diameter of the annular protruding portion, so the rod is extracted, after use, thereby the needle seat is possibly kept from the container portion and stored in the round tube of the syringe tube so as to prevent others from being stabbed by a discarded needle.

## Description

### BACKGROUND OF THE INVENTION

### 1. Field of the Invention

This invention relates to a safe syringe and particularly to a safe syringe forcing a used needle to be drawn back to the syringe in an injection tube.

### 2. Description of the Prior Art

Conventional needle of a syringe is mounted by rotation, and thus after using the needle, a user must use a lid to cover the needle and then discards it, thereby preventing others from being injured or infected due to a sting of the needle.

However, having used off the lid to cover the used needle, the user frequently stabs and wounds himself/herself due to a fit of giddiness, slip out of hands, or low concentration of spirits, whereas the stabbed user stabbed with the needle used by a patient suffering from hepatitis, AIDS, or the like caught via blood may be infected with the disease.

Consequently, in consideration of prevention of the defect of syringe stabbing the nursing staff who most often uses and then causing the staff to be infected, this inventor further improves it and finally successfully develops the safe syringe according to this invention after having made extraordinarily painstaking efforts to study the syringe diligently with a quiet mind for many years.

### SUMMARY OF THE INVENTION

This invention is to provide a safe syringe, a simplified syringe of reduced volume for achievement of environmental protection and waste disposal.

This invention is further to provide a used safe syringe of which a needle lid may be inserted into a syringe tube.

This invention is next to provide a used safe syringe of which a needle seat may be allowed in a container portion of the syringe tube.

This invention is again to provide a push rod of which a body and a neck are connected together through several connections and functions as a safe syringe of which the body and the neck may be broken off after the syringe is used.

In order to achieve the purposes mentioned above, the safe syringe comprises a syringe tube, a push rod, a needle seat and a needle lid.

The syringe tube has a top and a bottom. An annular protruding portion is provided adjacently around an inside of the bottom of the tube to form a container portion between the annular protruding portion and the bottom. The container portion is converged from a back end of the annular protruding portion along the bottom to form a cone shape and a piercing hole is formed in the bottom of the syringe tube. A bore diameter of the piercing hole is decreased from the container portion to the exterior of the bottom to form a cone shape.

The push rod is formed with a pressing portion, a body, a neck, a protruding pillar and a toggle. A front end of the neck is provided with an annular recess and the toggle is set on the annular recess. The protruding pillar has a cone shape with a round end.

The needle seat is provided with a base of which a recess with a curved surface is formed inside, a socket, and a connection portion. A top plane of the base has an oval shape with a major axis and a minor axis. A total length of a surrounding exterior of the top plane is less than that of a surrounding interior of the annular protruding portion. A bottom plane of the base has a circular shape and is opened with a circular aperture extending from the recess through the socket to the connection portion so that a needle can be put into the connection portion to connect the aperture.

The needle lid is in the form of a hollow cone to cover the needle. There are several raised ribs provided around the exterior of the needle lid.

### BRIEF DESCRIPTION OF THE DRAWINGS

The drawings disclose an illustrative embodiment of the present invention which serves to exemplify the various advantages and objects hereof, and are as follows:
Fig. 1 is an exploded view of a first embodiment of a safe syringe according to this invention;
Fig. 2 is an exploded view of the second embodiment of the safe syringe according to this invention;
Fig. 3 is an exploded, perspective view of the safe syringe in Fig. 1;
Fig. 4 is a partially enlarged view of a syringe tube combined with a needle seat;
Fig. 5 is a partially schematic view illustrating a push rod provided in the syringe tube;
Fig. 6 is an assembly view of the safe syringe;
Fig. 7 is an enlarged assembly view of the safe syringe;
Fig. 8 is a schematic view illustrating a needle seat kept from a container portion after a cone of the push rod is combined with a joint of the base; and
Fig. 9 is a schematic view illustrating the push rod of the safe syringe extracted from a syringe tube and illustrating a body and a neck of the push rod that are broken off.

### DETAILED DESCRIPTION OF THE PREFERRED EMBODIMENT

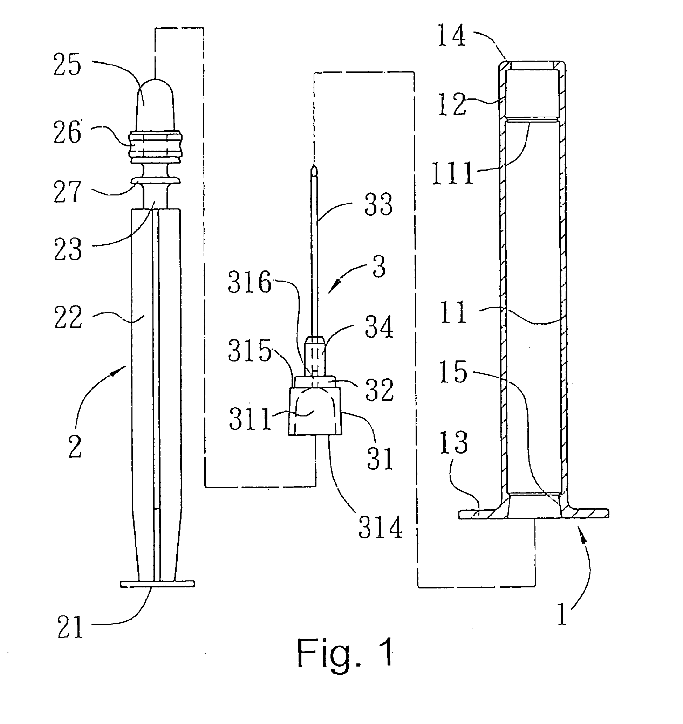

Referring now to Figs. 1, 2, and 3, a safe syringe according to this invention comprises a syringe tube 1, a push rod 2, a needle seat 3 and a needle lid 4.

A syringe tube 1 includes a round tube 11 having a top and a bottom ends. An annular protruding portion 111 is provided adjacently around an inside of the bottom of the tube 11 to form a container portion 12 between the annular protruding portion 111 and the bottom end. The container portion 12 has slightly decreased internal diameter from a back end of the annular protruding portion 111 along the bottom of the tube 11 to form a cone shape. A piercing hole 14 is formed in the bottom end of the tube 11. A bore diameter of the piercing hole 14 is decreased from the container portion 12 to the exterior of the bottom to form a cone shape.

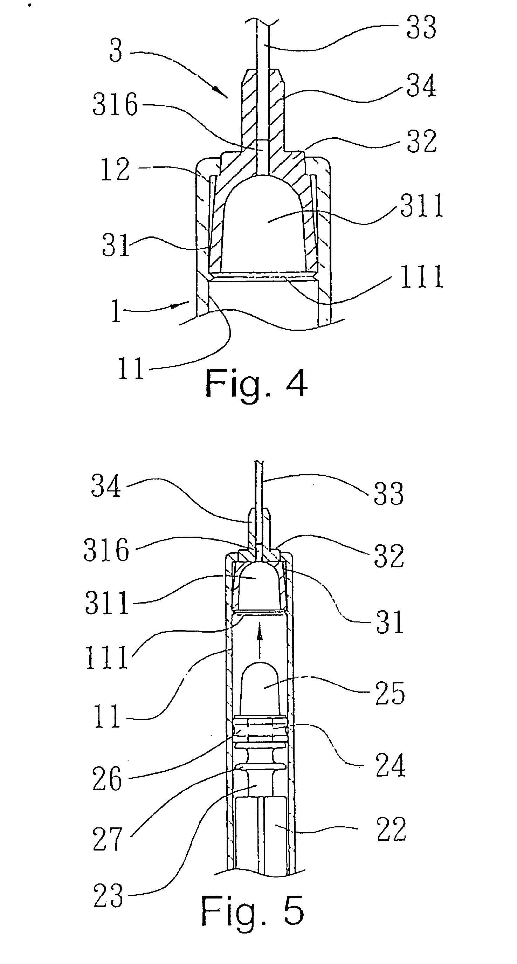

The push rod 2 is formed with a pressing portion 21, a body 22, a neck 23, a protruding pillar 25, and a toggle 26. A front end of the neck 23 is provided with an annular recess 24 (as shown in Fig. 5) and the toggle 26 is set on the annular recess 24. The toggle 26 can be made of a rubber or a silica gel. In this embodiment the toggle 26 is made of the silica gel and the protruding pillar 25 is formed as a cone shape with a round end.

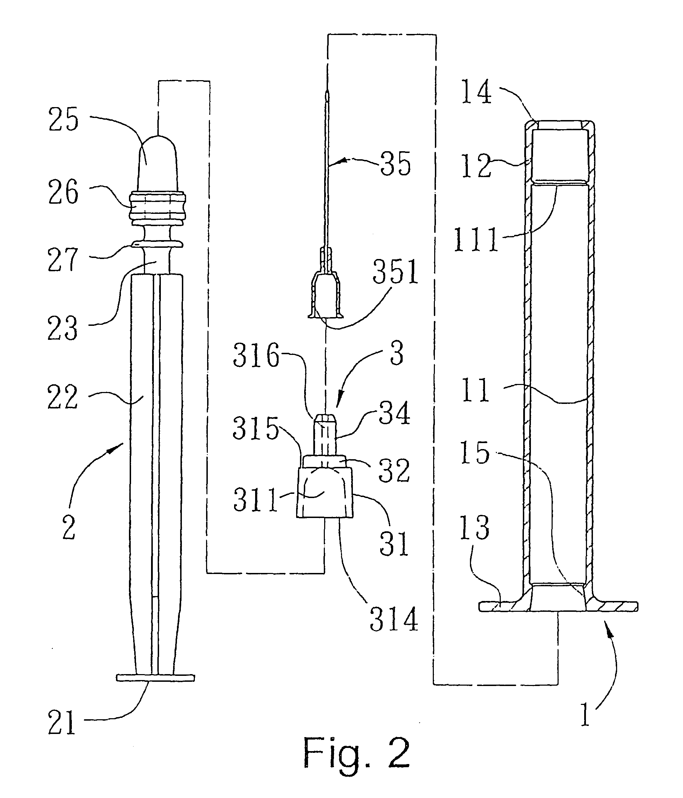

The needle seat 3 is provided with a base 31 of which a recess 311 with a curved surface is formed inside to securely fit the protruding pillar 25, a socket 32, and a connection portion 34. A top plane 314 of the base 31 has an oval shape with a major axis 312 and a minor axis 313. A total length of a surrounding exterior of the top plane 314 is less than that of a surrounding interior of the annular protruding portion 111. A bottom plane 315 of the base 31 has a circular shape and is opened with a circular aperture 316 extending from the recess 311 through the socket 32 to the connection portion 34 so that a needle 33 can be put into the connection portion 34, as shown in Fig. 1, to connect the aperture 316. Or, in another embodiment, as shown in Fig. 2, the needle 33 includes a mounting seat 35 to be mounted on the connection portion 34 by a fixing joint 351 so that the needle 33 is connected to the aperture 316.

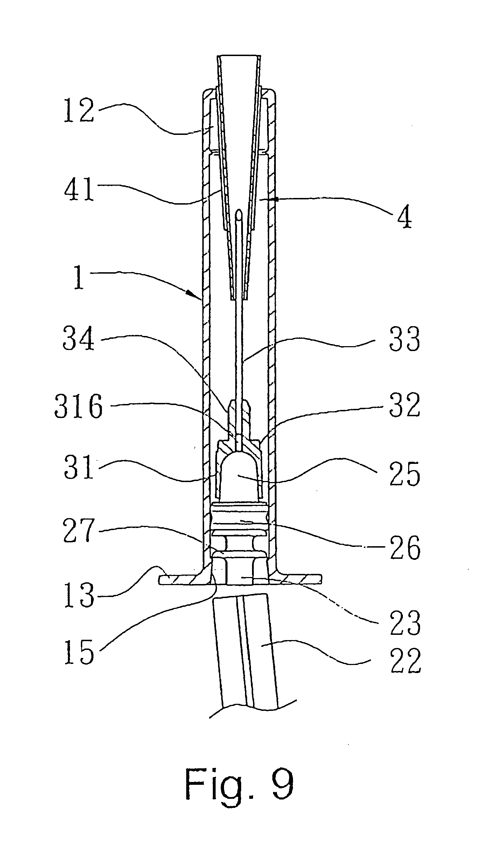

The needle lid 4 is in the form of a hollow cone to cover the needle 33, which can be combined with the syringe tube 1 after the used needle 33 is drawn into the tube 11 as shown in Fig. 9. There are several raised ribs 41 provided around the exterior of the needle lid 4. The lid 4 can provide an opening on the top or to be stabbed by the needle 33 as shown in Fig. 9.

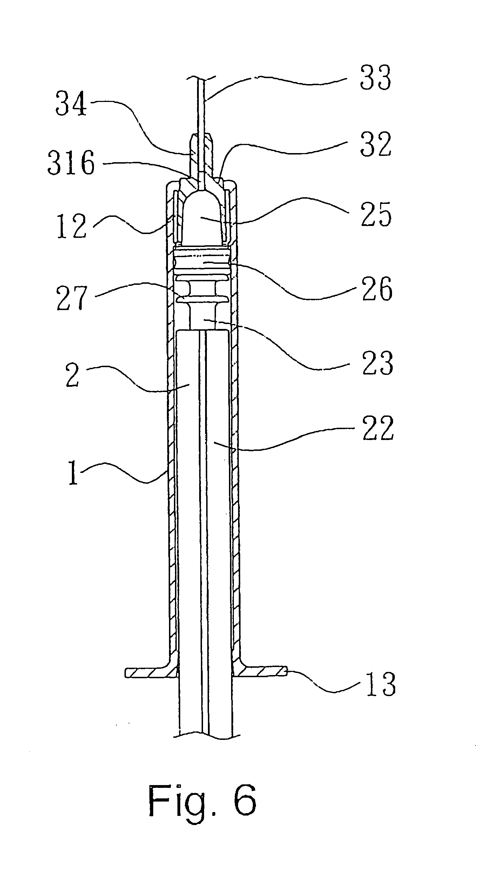

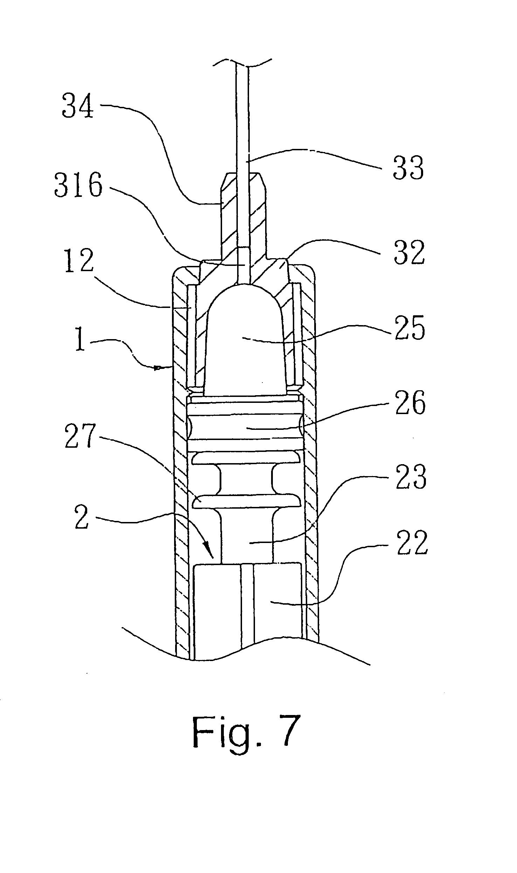

Referring now to Fig. 4, the needle seat 3 is put into the round tube 11 through the piercing hole 14 with the base 31 pushing against the annular protruding portion 111 by the oval plane 314 and the socket fitting in the piercing hole 14 so as to install the base 31 in the container portion 12. Meanwhile, the toggle 26 is fitted in the round tube 11 so that the pushing rod 2 is movable inside the tube 11. After injection of a medicament, a protruding pillar 25 on the rod 2 will fixedly combine in the recess 311 of the base 31 by a pressing and a frictional force, as shown in Fig. 6. That is, when the user push the rod 2 to inject the medicament, as shown in Fig. 5, the protruding pillar 25 will force the curved surface of the recess 311 to have the pillar 25 tightly fit with the base 31, as shown in Fig. 7. Because the external diameter of the top plane 314 of the base is slightly smaller than the internal diameter of the annular protruding portion 111, when the user pulls the push rod 2, the needle seat 3 can be drawn from the container portion 12 to have the needle 33 rested in the round tube 11.

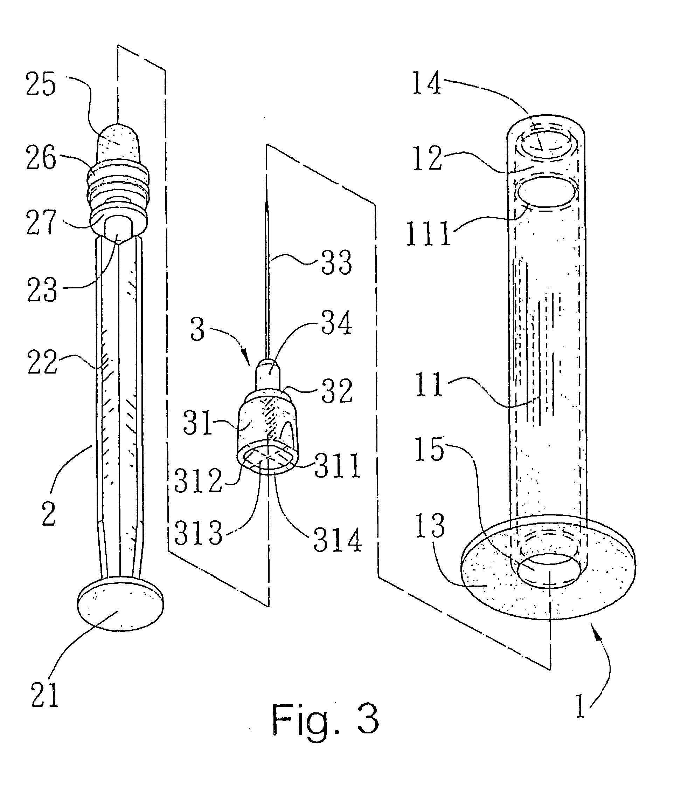

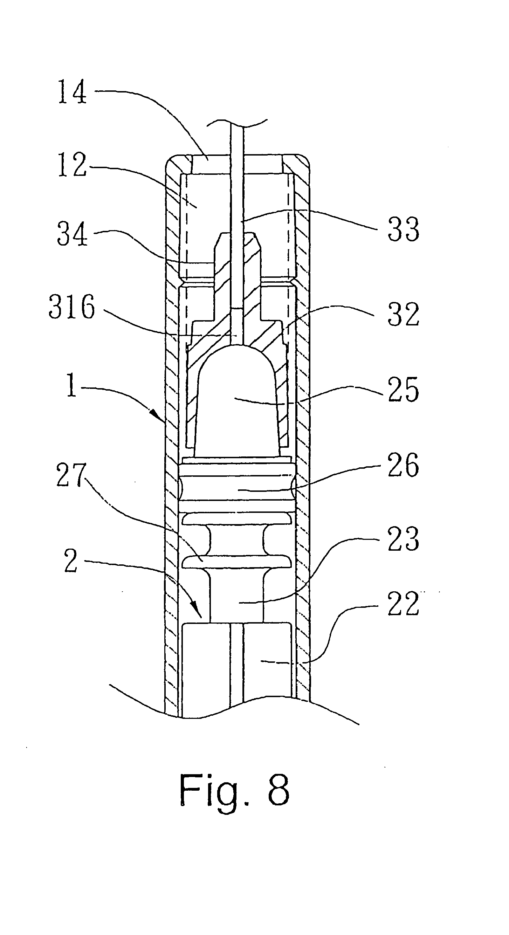

For more details, referring again to Fig. 3 to Fig. 9, when using the safe syringe of the present invention, a forefinger and a middle finger are used to handle on a fringe 13 of the syringe tube 1 and a thumb is used to press on the rear end 21 of the push rod 2 so that the medicament contained in the tube 11 can be injected to the body through the needle 33. When the the push rod moves to the bottom of the tube 11, the protruding pillar 25 of the push rod 2 is combined in the recess 311 of the base 31. Thereby the oval base 31 is transformed into a circular base 31 and a diameter of the circular base 31 is slightly smaller than a bore diameter of the annular protruding portion 111. Thus, when the user pulls the push rod 2 back, the needle seat 3 is moved from the container portion 12 of the syringe tube 1 due to backward extraction of the push rod 2 and the needle 33 is drawn back inside the round tube 11.

Furthermore, the exterior of the needle lid 4 with several raised ribs 41 makes the needle lid 4 to be securely inserted in the piercing hole 14.

In the syringe tube 1 according to this invention, the piercing hole 14 connects the container portion 12 of which the bore diameter is larger than that of the piercing hole 14, thereby the container portion 12 being in the form of topography; thus, when being provided in the syringe tube 1, the base 31 may be fully corresponding to the container portion 12 and the piercing hole 14 and may be thoroughly allowed in the container portion 12, and thereby the needle seat 3 cannot get out of the syringe tube 1 through the piercing hole 14; both the container portion 12 and the piercing hole 14 designed in the form of topography may also prevent leakage of the medicament when syringed.

Again, the fringe 13 of the syringe tube 1 are provided with tilting portions 15, and a joint between the tilting portion 15 and a penetration hole 11 is in the form of topography; thus, when the safe syringe is used off and the user moves the push rod 2 to the fringe 13, a stopper portion 27 of the push rod may stop at the fringe 13 so that the push rod 2 cannot move to depart from the syringe tube 1, and further several connections between the body 22 and neck 23 of the push rod 2 connect to each other, so the body and 22 the neck 23 may be broken off after the syringe tube 1 is used off.

In comparison with the case cited above as proof and other conventional skills, the safe syringe according to this invention further has the following advantages:
(1) When the push rod is pushed to the bottom of syringe tube, the needle seat may be extracted from the container portion according to the push rod and may be allowed in the round tube for disposal of the waste and prevention of others from being stabbed by the needle.
(2) When the syringe is used off, the needle lid may be inserted into the piercing hole of the base and then may altogether be dumped in consideration of a physical volume at the time of recycling execution.

Many changes and modifications in the above described embodiment of the invention can, of course, be carried out without departing from the scope thereof. Accordingly, to promote the progress in science and the useful arts, the invention is disclosed and is intended to be limited only by the scope of the appended claims.

## Claims

1. A safe syringe comprising:
a syringe tube having a top and a bottom, an annular protruding portion being provided adjacently around an inside of the bottom of the tube to form a container portion between the annular protruding portion and the bottom;
a push rod formed with a pressing portion, a body, a neck, a protruding pillar and a toggle, a front end of the neck being provided with an annular recess and the toggle being set on the annular recess;
a needle seat provided with a base of which a recess with a curved surface is formed inside, a socket, and a connection portion, wherein there is a circular aperture extending from the recess through the socket to the connection portion so that a needle can be put into the connection portion to connect the aperture; and
a needle lid in the form of a hollow cone to cover the needle.

2. The safe syringe according to claim 1, wherein the push rod of which a body and a neck are connected together through several connections and the body and the neck may be broken off after the syringe is used.

3. The safe syringe according to claim 1, wherein the toggle is made of a rubber or a silica gel.

4. The safe syringe according to claim 1, wherein the needle lid after used may be securely placed in the piercing hole of the syringe tube for reduction of a recycling volume.

5. The safe syringe according to claim 1, wherein the circular aperture provided in the connection portion of needle seat may be mounted with a needle.

6. The safe syringe according to claim 1, wherein the connection portion of needle seat may be provided with a needle seat.
